# EUROPÄISCHE PATENTSCHRIFT

(11) **EP 1 261 581 B1**
(45) Veröffentlichungstag und Bekanntmachung des Hinweises auf die Patenterteilung: **24.05.2006**
(21) Anmeldenummer: 01901150.1
(22) Anmeldetag: 12.01.2001
(51) Int. Cl.: C07C 279/36

(54) **VERFAHREN ZUR HERSTELLUNG VON 1-METHYL-3-NITROGUANIDIN**
METHOD FOR PRODUCING 1-METHYL-3-NITROGUANIDINE
PROCEDE DE PRODUCTION DE 1-METHYL-3-NITROGUANIDINE

(30) Priorität: 28.01.2000 DE 10003834
(43) Veröffentlichungstag der Anmeldung: 04.12.2002
(73) Patentinhaber: NIGU CHEMIE GMBH, D-84478 Waldkraiburg (DE)
(72) Erfinder: KERN, Norbert, 45721 Haltern am See (DE)
(74) Vertreter: Klusmann, Peter
(86) Internationale Anmeldenummer: PCT/EP2001/000351
(87) Internationale Veröffentlichungsnummer: WO 2001/055099

(56) Entgegenhaltungen:
- EP-A- 0 798 293
- WO-A-01/51458
- MCKAY A F ET AL: "PREPARATION AND PROPERTIES OF N-METHYL-N-NITROSO-N'-NITROGUANIDINE" JOURNAL OF THE AMERICAN CHEMICAL SOCIETY,US,AMERICAN CHEMICAL SOCIETY, WASHINGTON, DC, Bd. 69, 1. Dezember 1947 (1947-12-01), Seiten 3028-3030, XP002033022 ISSN: 0002-7863 in der Anmeldung erwähnt

## Beschreibung

### Technisches Gebiet

Die vorliegende Erfindung betrifft ein Verfahren zur Herstellung von 1-Methyl-3-nitroguanidin, welches ein wichtiges Zwischenprodukt zur Herstellung von biologisch aktiven Verbindungen, insbesondere von Insektiziden darstellt (vgl. "Nicotinoid Insecticides and the Nicotinic Acetylcholine Receptor", I.Yamamoto, J.E. Casida (Ed.) Springer-Verlag, Tokyo 1999).

### Stand der Technik

Für die Herstellung von 1-Methyl-3-nitroguanidin sind entsprechend dem Stand der Technik schon eine Reihe von Vorschlägen gemacht worden. Eine bekannte Herstellungsmethode stellt hierbei die Dehydratisierung von Methylguanidinnitrat mit Hilfe von Schwefelsäure dar (vgl. J. Amer. Chem. Soc. 1933, 55, 731). Das entsprechende Methylguanidinnitrat ist zwar gemäß US 2,425,341 aus wässriger Cyanamid-Lösung und Methylammoniumnitrat zugänglich, doch ist das Verfahren wegen der relativ geringen Ausbeute und der erforderlichen Nachreinigung unbefriedigend.

Entsprechend der in der WO 98/43 951 beschriebenen Verfahrensvariante wird Methylguanidinnitrat durch Umsetzung von Methylammoniumnitrat mit wasserfreiem Cyanamid in organischen Lösemitteln wie Ether oder Alkoholen erhalten und danach direkt zu 1-Methyl-3-nitroguanidin umgesetzt. Die Verwendung wasserfreien Cyanamids ist jedoch im Vergleich zur wässrigen Cyanamid-Lösung unwirtschaftlich.

Des Weiteren ist bekannt, dass man 1-Methyl-3-nitroguanidin durch Nitrierung von Methylguanidinsulfaten erhalten kann (vgl. J. Amer. Chem. Soc. 1933, 55, 731). Auch diese Herstellungsmethode hat den Nachteil einer nicht zufriedenstellenden Ausbeute sowie einer relativ großen Abwassermenge.

Auch die Umsetzung von Methylammoniumnitrat mit Calciumcyanamid oder Dicyandiamid (vgl. Can. J. Chem. 1958, 36, 737) führt zu unbefriedigenden Ergebnissen, da die Reinheit des so erhaltenen Methylguanidinnitrats weniger als 90 % beträgt.

Ein weiteres Verfahren zur Herstellung von 1-Methyl-3-nitroguanidin erfolgt in der Weise, dass man zunächst Methylisothioharnstoffsulfat nitriert und anschließend die Methylmercaptogruppe gegen einen Methylamino-Rest substituiert (vgl. J. Amer. Chem. Soc. 1954, 76, 1877). Dabei wirft die Abspaltung von Methylmercaptan, vor allem bei einer Durchführung im großtechnischen Maßstab, erhebliche verfahrenstechnische Probleme auf.

Außerdem ist bekannt, dass man 1-Methyl-3-nitroguanidin dadurch erhalten kann, dass man eine stark alkalische Nitroguanidin-Lösung mit einem großen Überschuss an Methylammoniumchlorid bei 60 °C umsetzt (vgl. J. Amer. Chem. Soc. 1947, 69, 3028 und J. Chem. Soc. 1957, 70). Bei diesem Prozess sind die Reinheit der Endprodukte, die in aufwendigen Verfahren gereinigt werden müssen, und insbesondere die erzielten Ausbeuten zwischen 43 und 66 % an reinem Produkt sehr unbefriedigend. Als Nebenprodukte entstehen unter Entwicklung von gasförmigem N₂O sehr große Mengen an Harnstoff- bzw. Guanidin-Derivaten. Außerdem entstehen hierbei große Mengen an Abwasser.

Auch die direkte Umsetzung von wässrigen Methylamin-Lösungen mit Nitroguanidin bei Temperaturen > 60 °C (vgl. J. Amer. Chem. Soc. 1927, 49, 2304 und Proc. Am. Acad. Arts Sci. 1926, 61, 437) führt nur in ungenügenden Ausbeuten von 36 bis 45 % zu 1-Methyl-3-nitroguanidin.

Dieselben Nachteile weist auch die in der EP-A 798 293 beschriebene direkte Umsetzung von Methylamin-Lösung mit Nitroguanidin bei 0 - 40 °C auf. Auch bei dieser Vorgehensweise wird das gewünschte Endprodukt nur in max. Ausbeuten von 60 % erhalten. Das Verfahren ist außerdem sehr unwirtschaftlich aufgrund der langen Reaktionszeiten von ca. 24 h und der hohen Verdünnung, die neben ungenügenden Raum/Zeit-Ausbeuten zu sehr großen Abwassermengen führt.

Die WO 01/51458 (veröffentlicht am 19. Juli 2001) offenbart ein Verfahren zur Herstellung von 1-Methyl-3-nitroguanidin durch Umsetzung von Nitroguanidin mit wässriger Methylaminlösung, die durch Zugabe von anorganischer oder organischer Säure gepuffert wurde. Die Verwendung eines Methylammonium-Salzes anstelle von Methylamin und die pH-Einstellung mit Base werden jedoch nicht erwähnt.

### Darstellung der Erfindung

Der vorliegenden Erfindung lag daher die Aufgabe zugrunde, ein Verfahren zur Herstellung von 1-Methyl-3-nitroguanidin zu entwickeln, welches die Nachteile des Standes der Technik nicht aufweist, sondern das gewünschte Endprodukt in sehr guten Ausbeuten und hohen Reinheiten auf besonders umweltschonende und technisch einfache Weise ermöglicht.

Diese Aufgabe wurde erfindungsgemäß dadurch gelöst, dass man Nitroguanidin mit einem Methylammonium-Salz in wässriger Lösung bei Temperaturen von 30 bis 60 °C und bei einem pH-Wert von 9,5 bis 12,3 umsetzt, wobei die pH-Wert-Einstellung mit Hilfe von Methylamin und/oder einer starken Base vorgenommen wird. Es hat sich hierbei überraschenderweise gezeigt, dass neben der Ausbeutesteigerung auch die Bildung von N₂O erheblich reduziert wird, wodurch die Gefahr der Bildung explosionsfähiger Gemische mit dem als Nebenprodukt gebildeten Ammoniak erheblich vermindert wird.

Beim Verfahren entsprechend der vorliegenden Erfindung wird also Nitroguanidin mit einem Methylammonium-Salz in wässriger Lösung bei Temperaturen von 30 bis 60 °C, insbesondere bei 40 bis 45 °C, umgesetzt.

Als Methylammonium-Salze können hierbei die Salze von starken Mineralsäuren, wie z. B. das Sulfat, Hydrochlorid, Nitrat oder Phosphat verwendet werden.

Es ist als erfindungswesentlich anzusehen, dass der pH-Wert während der Umsetzung auf einen Wert von 9,5 bis 12,3 eingestellt wird. Überraschenderweise wurde festgestellt, dass die erzielbare Ausbeute sehr stark vom pH-Wert während der Umsetzung abhängt. Bei der Nacharbeitung der Verfahren entsprechend dem Stand der Technik wurde festgestellt, dass dabei pH-Werte von 13,0 bis 14,0 auftreten, die stets oberhalb des pKs-Wertes von Nitroguanidin (12,4) liegen. Bei solchen Reaktionsbedingungen sind bedingt durch Nebenreaktionen Ausbeuten deutlich oberhalb von 60 % nicht zu erreichen. Wird die Reaktion dagegen bei einem pH-Wert durchgeführt, der unterhalb des pKs-Wertes von Nitroguanidin liegt, werden Ausbeuten in Höhe von 80 bis 95 % erhalten. Als besonders vorteilhaft haben sich hierbei pH-Werte von 11,0 bis 12,0 während der Umsetzung erwiesen. Bei pH-Werten, die deutlich unter 9,5 liegen, sinkt die Ausbeute wieder.

Die Einstellung des pH-Wertes erfolgt entweder durch Zugabe des Methylamins und des Methylammonium-Salzes in solchen Molverhältnissen, dass sich der gewünschte pH-Wert von selbst einstellt, oder durch den üblichen Einsatz starker Basen. Als starke Basen werden bspw. Natronlauge oder Kalilauge verwendet. Gemäß einer bevorzugten Ausführungsform wird/werden das Methylamin und/oder das Methylammonium-Salz in einer Menge eingesetzt, dass die molare Menge der Summe der beiden Komponenten, bezogen auf das eingesetzte Nitroguanidin, einem Überschuss von 5 bis 50 mol-% entspricht.

Prinzipiell kann auch ein höherer Überschuss an Methylamin bzw. Methylammonium-Salz mit bis zu 100 % verwendet werden, ohne dass hierbei eine wesentliche Bildung des dialkylierten Produkts 1,2-Dimethyl-3-nitroguanidin beobachtet wird. Ein höherer Überschuss als die bevorzugten 5 bis 50 Gew.-% an Methylamin bzw.
Methylammonium-Salz mindert jedoch die Wirtschaftlichkeit des erfindungsgemäßen Verfahrens.

Nach Abschluss der Umsetzung, die in der Regel nach 2 bis 3 Stunden beendet ist, wird das Endprodukt vorzugsweise durch Filtration abgetrennt. Eine Neutralisation des Reaktionsgemisches vor der Filtration ist ebenfalls möglich, ohne dass sich dies in irgendeiner Form negativ auf die Ausbeute auswirkt. Ggf. kann das Endprodukt mit etwas kaltem Wasser nachgewaschen und getrocknet werden.

Die Vorteile des erfindungsgemäßen Verfahrens bestehen im Wesentlichen darin, dass man 1-Methyl-3-nitroguanidin in Ausbeuten von mindestens 80 % und Reinheiten > 99 % auf besonders umweltschonende und technisch einfache Weise erhalten kann, weshalb sich dieses Verfahren besonders für den großtechnischen Einsatz eignet.

Die nachfolgenden Beispiele sollen die Erfindung näher veranschaulichen.

### Beispiele

### Beispiel 1

100 g (0,63 mol) Methylammoniumsulfat werden in 400 ml H₂O gelöst. Mit ca. 53 g (0,66 mol) 50 % Natronlauge wird ein pH-Wert von 11,5 eingestellt. In diese Lösung werden bei 40 bis 42 °C 100 g (0,96 mol) Nitroguanidin eingerührt. Nach 3 h Rühren bei 40 bis 42 °C wird das Reaktionsgemisch bei max. 30 °C neutralisiert. Nach dem Abkühlen auf 5 °C wird die Suspension abfiltriert, mit 120 ml kaltem H₂O gewaschen und getrocknet. Man erhält 108 g Methylnitroguanidin (Gehalt > 99 % per HPLC, Schmp.: 158 - 159 °C) entsprechend einer Ausbeute von 95 %.

### Beispiel 2

Unter starkem Rühren werden zu einem Gemisch aus 2,65 kg (16,5 mol) Methylammoniumsulfat und 15,4 kg (165 mol) 40 %-Methylaminlösung in 41 1 H₂O 19,8 kg (152 mol) Nitroguanidin mit einem Wassergehalt von 20 Gew.-% bei 40 - 42 °C gegeben, wobei das Reaktionsgemisch einen pH-Wert von 11,3 aufweist. Nach 2 h wird das Reaktionsgemisch auf 3 °C gekühlt, abfiltriert, 3 mal mit je 6 1 gekühltem H₂O gewaschen und getrocknet. Man erhält 14,8 kg Methylnitroguanidin (Gehalt > 99 % per HPLC, Schmp.: 159 °C) entsprechend einer Ausbeute von 82 %.

## Patentansprüche

1. Verfahren zur Herstellung von 1-Methyl-3-nitroguanidin, **dadurch gekennzeichnet, dass** man Nitroguanidin mit einem Methylammonium-Salz in wässriger Lösung bei Temperaturen von 30 bis 60 °C und bei einem pH-Wert von 9,5 bis 12,3 umsetzt, wobei die pH-Wert-Einstellung mit Hilfe von Methylamin und/oder einer starken Base vorgenommen wird.

2. Verfahren nach Anspruch 1, **dadurch gekennzeichnet, dass** man die Umsetzung bei einem pH-Wert von 11,0 bis 12,0 durchführt.

3. Verfahren nach den Ansprüchen 1 oder 2, **dadurch gekennzeichnet, dass** man als Methylammonium-Salz das entsprechende Sulfat, Hydrochlorid, Nitrat oder Phosphat verwendet.

4. Verfahren nach einem der Ansprüche 1 bis 3, **dadurch gekennzeichnet, dass** man als starke Basen Natronlauge oder Kalilauge heranzieht.

5. Verfahren nach einem der Ansprüche 1 bis 4, **dadurch gekennzeichnet, dass** man das Methylammonium-Salz evtl. zusammen mit Methylamin in einer Menge einsetzt, so dass die molare Menge der Summe der beiden Komponenten, bezogen auf das Nitroguanidin , einem Überschuss von 5 bis 50 mol-% entspricht.

6. Verfahren nach einem der Ansprüche 1 bis 5, **dadurch gekennzeichnet, dass** man die Umsetzung bei einer Temperatur von 40 bis 45 °C durchführt.

## Claims

1. Process for the production of 1-methyl-3-nitroguanidine, **characterised in that** nitroguanidine is reacted with a methylammonium salt in aqueous solution at temperatures of 30 to 60°C and at a pH value of 9.5 to 12.3, wherein the pH value adjustment is carried out with the aid of methylamine and/or a strong base.

2. Process according to claim 1, **characterised in that** the reaction is carried out at a pH value of 11.0 to 12.0.

3. Process according to claims 1 or 2, **characterised in that** the corresponding sulphate, hydrochloride, nitrate or phosphate is used as the methylammonium salt.

4. Process according to one of claims 1 to 3, **characterised in that** sodium hydroxide solution or potassium hydroxide solution is used as the strong base.

5. Process according to one of claims 1 to 4, **characterised in that** the methylammonium salt, possibly together with methylamine, is used in a quantity so that the molar quantity of the sum of the two components, relative to the nitroguanidine, corresponds to an excess of 5 to 50 mole%.

6. Process according to one of claims 1 to 5, **characterised in that** the reaction is carried out at a temperature of 40 to 45°C.

## Revendications

1. Procédé de production de 1-méthyl-3-nitroguanidine, **caractérisé en ce que** l'on convertit la nitroguanidine avec un sel de méthylammonium en solution aqueuse, à des températures dans la fourchette de 30 à 60°C et à une valeur de pH dans la fourchette de 9,5 à 12,3, le réglage de la valeur de pH étant effectué à l'aide de méthylamine et/ou d'une base forte.

2. Procédé selon la revendication 1, **caractérisé en ce que** l'on effectue la conversion à une valeur de pH dans la fourchette de 11,0 à 12,0.

3. Procédé selon les revendications 1 ou 2, **caractérisé en ce qu'**on utilise comme sel de méthylammonium le sulfate, l'hydrochlorure, le nitrate ou le phosphate correspondant.

4. Procédé selon l'une des revendications 1 à 3, **caractérisé en ce qu'**on utilise comme base forte la lessive de soude ou la lessive de potassium.

5. Procédé selon l'une des revendications 1 à 4, **caractérisé en ce que** l'on utilise le sel de méthylammonium, éventuellement conjointement avec la méthylamine, en une quantité telle que la quantité molaire de la somme des deux composants, en se référant à la nitroguanidine, corresponde à un excès de 5 à 50 mol %.

6. Procédé selon l'une des revendications 1 à 5, **caractérisé en ce qu'**on effectue la conversion à une température dans la fourchette de 40 à 45°C.
